# EUROPEAN PATENT SPECIFICATION

(11) **EP 2 505 226 B1**
(45) Date of publication and mention of the grant of the patent: **29.05.2013**
(21) Application number: 12159650.6
(22) Date of filing: 15.03.2012
(51) Int. Cl.: A61M 25/09

(54) **Guidewire**
Führungsdraht
Fil-guide

(30) Priority: 31.03.2011 JP 2011076947
(43) Date of publication of application: 03.10.2012
(73) Proprietor: Asahi Intecc Co., Ltd., Nagoya-shi, Aichi 463-0024 (JP)
(72) Inventor: Matsuo, Kenichi, Nagoya-shi, Aichi 463-0024 (JP)
(74) Representative: Winter, Brandl, Fürniss, Hübner, Röss, Kaiser, Polte - Partnerschaft

(56) References cited:
- JP-A- 2007 135 645
- US-A1- 2005 065 456
- US-A1- 2008 045 908
- US-A1- 2009 118 644

## Description

### BACKGROUND OF THE INVENTION

### 1. Field of the Invention

The present invention relates to a guidewire.

### 2. Description of the Related Art

Guidewires are known as medical mechanical devices used to guide a device such as a balloon or a stent to a lesion in percutaneous transluminal coronary angioplasty (PTCA).

JP 2007-135645 A discloses an example of such a guidewire which includes a core shaft including a front end portion and a rear end portion, a helical coil that covers the front end portion of the core shaft, a solder part that bonds the core shaft and the helical coil together, and a resin coating layer formed at the surface of the helical coil.

The front end portion and the rear end portion of the core shaft serve as a distal portion and a proximal portion, respectively, of the guidewire. The distal portion of the guidewire is inserted into the body, and the proximal portion of the guidewire is operated by an operator, such as a doctor.

In the guidewire described in JP 2007-135645 A, the helical coil is formed of a wire having a roughened surface so that the resin coating layer tightly adheres to the helical coil.

However, in the guidewire according to the related art, the bonding strength between the helical coil and the solder part is not sufficiently high. Therefore, the helical coil is easily detached from the guidewire in response to an external force, such as a pulling force, when the guidewire is being used.

In particular, when the guidewire includes a helical coil formed of a wire including tungsten, it is relatively difficult to bond the helical coil with the solder part. Therefore, the bonding strength between the helical coil and the solder part in such a guidewire is even lower.

### SUMMARY OF THE INVENTION

Starting from JP 2007-135645 A the object of the present invention is to provide a guidewire in which a helical coil is strongly bonded to a solder part. This object is solved by a guidewire according to claim 1. A currently preferred embodiment thereof is subject-matter of dependent claim 2.

As a result of diligent studies conducted by the inventor of the present invention to solve the above-described problem, it has been found that when a helical coil formed of a wire with a plurality of grooves that extend in a certain direction is used, the helical coil can be prevented from becoming detached from the guidewire. Thus, the guidewire according to the present invention has been produced.

More specifically, a guidewire according to the present invention includes a core shaft having a front end portion and a rear end portion, a helical coil in which the front end portion is inserted, and a solder part with which the front end portion and the helical coil are bonded to each other. The helical coil is formed of a wire having a plurality of grooves in a surface of the wire, the grooves being formed at least along an axial direction of the wire. The plurality of grooves may include both grooves which are formed along the axial direction of the wire and grooves which are formed along the circumferential direction of the wire. In the latter case, it is preferred that more grooves are formed along the axial direction of the wire than along the circumferential direction of the wire.

The structure of the guidewire according to the present invention and effects of the guidewire will now be described in detail.

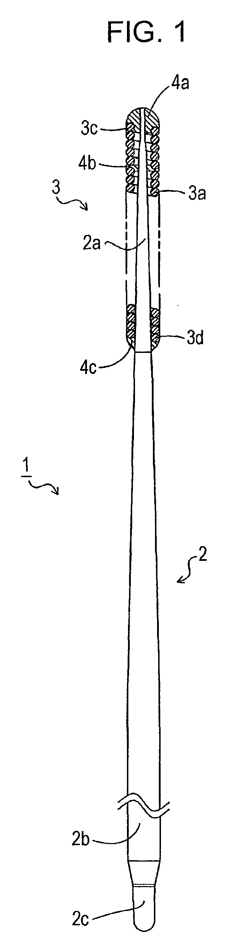

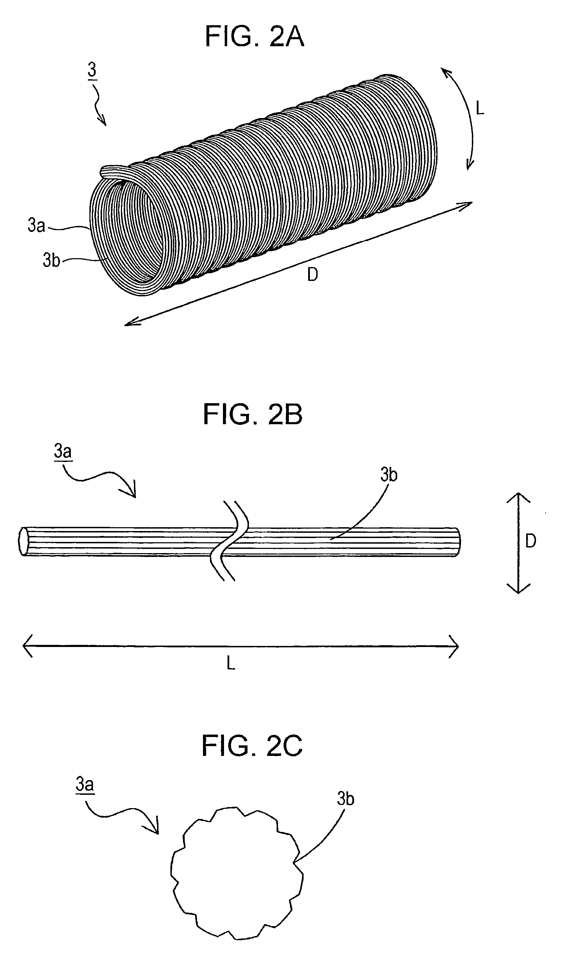

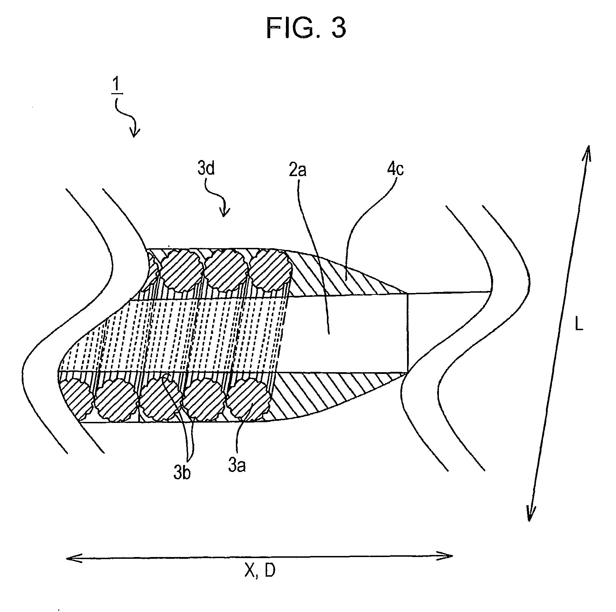

Fig. 1 is a schematic sectional view of a guidewire according to an embodiment of the present invention taken along a longitudinal direction of the guidewire. Fig. 2A is a schematic perspective view of a helical coil included in the guidewire illustrated in Fig. 1. Fig. 2B is a schematic perspective view of a wire obtained by stretching the helical coil illustrated in Fig. 2A. Fig. 2C is a schematic vertical sectional view of the wire illustrated in Fig. 2B taken along a plane perpendicular to the longitudinal direction of the wire. Fig. 3 is an enlarged sectional view of an area around a rear-end solder part in the guidewire illustrated in Fig. 1. In the following description, a distal portion of a guidewire and a front end portion of a core shaft are denoted by the same reference numeral, and a proximal portion of the guidewire and a rear end portion of the core shaft are denoted by the same reference numeral. In addition, a portion of the helical coil may be shown by broken lines, and detailed illustration of the portion of the helical coil may thus be omitted.

A guidewire 1 according to the present invention illustrated in Fig. 1 includes a core shaft 2 having a front end portion 2a and a rear end portion 2b, a helical coil 3 in which the front end portion 2a is inserted, and solder parts 4a, 4b, and 4c that bond the front end portion 2a and the helical coil 3 to each other. In the example illustrated in Fig. 1, the solder parts include a front-end solder part 4a, an intermediate solder part 4b, and a rear-end solder part 4c. However, the number of solder parts is not limited to three. For example, the solder parts may include only the front-end solder part and the rear-end solder part. Alternatively, two or more intermediate solder parts may be provided.

As illustrated in Figs. 2A, 2B, and 2C, a plurality of grooves 3b are formed in the surface of a wire 3a which forms the helical coil 3. In the embodiment shown in Figs. 2A, 2B, and 2C, the plurality of grooves includes only grooves formed along the axial direction of the wire. However, the plurality of grooves formed in the surface of the guidewire may further include (not shown) grooves formed along the circumferential direction. In this case, preferably more grooves 3b are formed along the axial direction L of the wire 3a than along a circumferential direction D of the wire 3a.

In this specification, when an imaginary straight line is drawn along the axial direction of the wire that forms the helical coil, grooves that are parallel to the imaginary straight line or grooves that are inclined in directions close to the imaginary straight line are called the grooves formed along the axial direction of the wire. In addition, when an imaginary perpendicular line that is perpendicular to the imaginary straight line is drawn, grooves that are parallel to the imaginary perpendicular line or grooves that are inclined in directions close to the imaginary perpendicular line are called the grooves formed along the circumferential direction of the wire. In addition, the state in which more grooves are formed along the axial direction of the wire than along the circumferential direction of the wire refers to the state in which the number of grooves formed along the axial direction of the wire is larger than the number of grooves formed along the circumferential direction of the wire. The directions along which the grooves are formed and the number of grooves can be determined by visual observation or observation using an optical microscope or a scanning electron microscope.

In the guidewire 1 according to the present invention in which the helical coil 3 having the above-described structure and the front end portion 2a of the core shaft 2 are bonded to each other by the solder parts 4a, 4b, and 4c, the grooves 3b in the helical coil 3 are filled with solder that forms the solder parts 4a, 4b, and 4c. Therefore, the helical coil 3 is strongly bonded with the solder parts 4a, 4b, and 4c as illustrated in, for example, Fig. 3, which is the enlarged view of the area around the rear-end solder part 4c.

In addition, as illustrated in Fig. 3, a longitudinal direction X of the guidewire 1 and the axial direction L of the wire 3a, along which the grooves 3b are mainly formed, cross at an angle close to about 90°. Therefore, even when an external force is applied in the longitudinal direction X of the guidewire 1, an anchoring effect against the external force is provided by the grooves formed along the direction substantially perpendicular to the direction in which the external force is applied and the solder with which the grooves are filled, i.e. by the grooves formed in the surface of the guidewire along the axial direction of the guidewire. Although the rear-end solder part 4c is explained above as an example, the helical coil 3 is also strongly bonded to the other solder parts, such as the front-end solder part 4a and the intermediate solder part 4b. Also at the other solder parts, the anchoring effect is provided by the grooves 3b and the solder with which the grooves 3b are filled.

With a guidewire according to the related art, when, for example, an operator pulls the guidewire to release the distal portion of the guidewire that has been caught by a lesion, the pulling force is applied mainly in the longitudinal direction of the guidewire. In such a case,the helical coil is easily detached from the guidewire.

In contrast, the helical coil 3 in the guidewire 1 according to the present invention is strongly bonded to the solder parts 4a, 4b, and 4c, owing to the grooves 3b formed mainly along a certain direction. Since the grooves 3b and the solder with which the grooves 3b are filled provide the anchoring effect, the helical coil 3 is not easily detached from the guidewire 1 even when a pulling force is applied to the guidewire 1 in the longitudinal direction X thereof. In case the plurality of grooves formed in the surface of the guidewire includes, in addition to the grooves formed along the axial direction of the wire, grooves formed along the circumferential direction of the wire, as above mentioned, the grooves formed along the circumferential direction of the wire may provide a certain anchoring effect against a relative rotation of the solder parts 4a, 4b, and 4c with respect to a guidewire that was not subjected to a roughening process and that lacks the grooves.

### BRIEF DESCRIPTION OF THE DRAWINGS

Fig. 1 is a schematic sectional view of a guidewire according to an embodiment of the present invention taken along a longitudinal direction of the guidewire.
Fig. 2A is a schematic perspective view of a helical coil included in the guidewire illustrated in Fig. 1.
Fig. 2B is a schematic perspective view of a wire obtained by stretching the helical coil illustrated in Fig. 2A.
Fig. 2C is a schematic vertical sectional view of the wire illustrated in Fig. 2B taken along a plane perpendicular to the longitudinal direction of the wire.
Fig. 3 is an enlarged sectional view of an area around a rear-end solder part in the guidewire illustrated in Fig. 1.
Fig. 4A is a scanning electron micrograph that shows an enlarged view of a tungsten wire forming a helical coil in a model of the guidewire according to the present invention.
Fig. 4B is a macrophotograph of a front end portion of the model including the tungsten wire illustrated in Fig. 4A.
Fig. 5A is a scanning electron micrograph that shows an enlarged view of a tungsten wire forming a helical coil in a model of a guidewire according to the related art.
Fig. 5B is a macrophotograph of a front end portion of the model including the tungsten wire illustrated in Fig. 5A.

### DESCRIPTION OF THE PREFERRED EMBODIMENTS

### First Embodiment

A first embodiment of a guidewire according to the present invention will be described with reference to the drawings. The guidewire according to the present embodiment has a structure similar to that of the above-described guidewire of the present invention. Therefore, explanations will be given with reference to Figs. 1 to 3. Explanations of matters similar to those of the above-described guidewire of the present invention may be omitted.

The guidewire 1 according to the present embodiment illustrated in Figs. 1 to 3 includes the core shaft 2 having the front end portion 2a and the rear end portion 2b, the helical coil 3 in which the front end portion 2a is inserted, and the front-end solder part 4a, the intermediate solder part 4b, and the rear-end solder part 4c which bond the front end portion 2a and the helical coil 3 to each other.

As described above with reference to Figs. 2A, 2B, and 2C, a plurality of grooves 3b are formed in the surface of the wire 3a which forms the helical coil 3, so that more grooves 3b are formed along the axial direction L of the wire 3a than along the circumferential direction D of the wire 3a. The structure of each component of the guidewire 1 according to the present embodiment will now be described.

### Structure of Core Shaft

The rear end portion 2b illustrated in Fig. 1 is tapered such that the diameter thereof decreases toward the front end of the core shaft 2. A connector portion 2c that provides connection to an extension guidewire or the like is provided at the rear end of the rear end portion 2b. The rear end portion 2b may instead have a cylindrical shape with a substantially constant diameter.

The front end of the rear end portion 2b is coupled to the front end portion 2a, which is tapered such that the diameter thereof decreases toward the front end of the core shaft 2.

Thus, the diameter of the core shaft 2 decreases toward the front end portion 2a from the rear end portion 2b. Therefore, the flexibility of the core shaft 2 increases toward the front end portion 2a from the rear end portion 2b.

The material of the core shaft 2 may be, for example, a stainless steel, a superelastic alloy such as Ni-Ti alloy, a piano wire, or a tungsten wire. The stainless steel may be, for example, a martensitic stainless steel, a ferritic stainless steel, an austenitic stainless steel, an austenitic-ferritic duplex stainless steel, or a precipitation hardened stainless steel. The material of the core shaft 2 is preferably a stainless steel, and more preferably, an austenitic stainless steel. In particular, SUS304, SUS316, or SUS316L is preferably used.

### Structure of Helical Coil

The helical coil 3 illustrated in Fig. 2A is formed into a tubular shape with a through hole at the center by helically winding a single wire 3a or a plurality of wires 3a.

The plurality of grooves 3b is formed in substantially the entire surface of each wire 3a that forms the helical coil 3. More grooves 3b are formed along the axial direction L of the wire 3a than along the circumferential direction D of the wire 3a.

The grooves 3b may have a maximum depth of, for example, 1.0 to 100 µm, and a length of, for example, 0.1 to 10 mm.

The material of the wire 3a may be, for example, a stainless steel such as a martensitic stainless steel, a ferritic stainless steel, an austenitic stainless steel, an austenitic-ferritic duplex stainless steel, or a precipitation hardened stainless steel, a superelastic alloy such as a Ni-Ti alloy, an X-ray impermeable metal such as platinum, gold, tungsten, tantalum, or iridium, or an alloy of the X ray impermeable metals. In particular, tungsten or a tungsten alloy is preferably used.

As illustrated in Fig. 1, the front end portion 2a is inserted in the helical coil 3 so that the helical coil 3 covers the front end portion 2a. The front end portion 2a is spaced by a predetermined distance from the inner wall of the helical coil 3.

The adjacent portions of the wire 3a are in contact with each other over the entire area of the helical coil 3 from a front end 3c to a rear end 3d. Therefore, the helical coil 3 has a substantially constant flexural property over the entire body thereof. In the area around the front end of the helical coil, pitches may be formed between the adjacent portions of the wire. In such a case, the flexibility of the helical coil is increased at the area around the front end thereof. In the area around the rear end of the helical coil, the wire may be densely wound such that the adjacent portions of the wire are in contact with each other. In such a case, the helical coil is not easily twisted even when a twisting force is applied to the helical coil in the area around the rear end thereof. As a result, a torque generated when the proximal portion of the guidewire is rotated can be efficiently transmitted to the distal portion of the guidewire.

The front end 3c of the helical coil 3 and the front end of the front end portion 2a are fixed to each other with the front-end solder part 4a, which has a hemispherical shape. The helical coil 3 and the front end portion 2a are fixed to each other with the intermediate solder part 4b at an intermediate position that is separated from the front end 3c toward the rear end 3d of the helical coil 3. The rear end 3d of the helical coil 3 and the rear end of the front end portion 2a are fixed to each other with the rear-end solder part 4c.

The material of the solder that forms the front-end solder part 4a, the intermediate solder part 4b, and the rear-end solder part 4c may be, for example, an aluminum alloy, Ag, Au, Zn, Sn-Pb alloy, Sn-Au alloy, Pb-Ag alloy, or Sn-Ag alloy. In particular, Au, Sn-Au alloy, or Sn-Ag alloy is preferably used. This is because these materials achieve high bonding strength at the solder parts.

### Method for Forming Guidewire

The guidewire according to the present embodiment may be manufactured by the following method.
(1) The core shaft is formed by forming a wire rod into the above-described shape by, for example, a taper cutting process or a pressing process.
(2) A wire for forming the helical coil is subjected to a roughening process. The roughening process may be performed by, for example, immersing the wire in a high-temperature alkaline solution, electropolishing the wire in an alkaline solution, or polishing the wire by reciprocating an abrasive paper with a certain grit size in the axial direction of the wire.

The estimated mechanisms of immersing the wire in a high-temperature alkaline solution and electropolishing are described below. The tungsten wire is formed by drawing of a thick tungsten wire, and therefore tungsten crystal grains contained in the wire are seemed to be likely to align on the axial direction of the wire. As a result, the grooves which are formed by solving the grains in the immersing or electropolishing process may contain more grooves formed along the axial direction and fewer grooves formed along the circumferential direction.

Selecting an appropriate roughening process allows to form only grooves which extend along the axial direction, or to form both grooves which extend along the axial direction and circumferential direction of the wire with the number of grooves formed in the axial direction being more than the number of grooves formed in the circumferential direction.
(3) The helical coil is formed by helically winding one or more wires that have been subjected to the roughening process.
(4) The front end portion of the core shaft is inserted into the helical coil, and the core shaft and the helical coil are soldered to each other at predetermined positions. Thus, the guidewire of the present embodiment is manufactured.

The effects of the guidewire according to the present embodiment will now be described.
(1) In the guidewire according to the present embodiment, a plurality of grooves is formed in the surface of the wire that forms the helical coil, and preferably such that more grooves are formed along the axial direction of the wire than along the circumferential direction of the wire. Therefore, the helical coil is strongly bonded with the solder parts, and an anchoring effect can be obtained by the grooves and the solder with which the grooves are filled. Therefore, due the grooves formed along the axial direction of the wire the helical coil is not easily detached from the guidewire even when a pulling force is applied to the guidewire in the longitudinal direction thereof. Further, grooves formed along the circumferential direction of the wire can prevent the helical coil from twisting relative to the guidewire.
(2) Even when the wire that forms the helical coil includes tungsten, which has a relatively low wettability to solder, the grooves can be filled with the solder and the helical coil can be strongly bonded with the solder parts.
   In particular, when the wire includes tungsten and the solder is Sn-Ag alloy, the above-described effects can be usefully exploited. In contrast, when the wire including tungsten does not have the above-described grooves, the wire and the solder repel each other and the helical coil cannot be easily bonded with the solder parts. As a result, the bonding strength is reduced.
(3) When the wire that forms the helical coil includes tungsten, which is relatively strong among the X-ray impermeable metals, the occurrence of breakage can be reduced. In addition, an inexpensive helical coil can be produced with low environmental load.

### Examples

### Experimental Example 1

As a model of the guidewire according to the present embodiment, Model 1 was manufactured by the following process.

That is, a stainless steel rod having the shape of the core shaft, a helical coil (tungsten coil), and Sn-Ag alloy solder were prepared. The stainless steel rod had a diameter of 0.081 mm and a length of 200 mm. The helical coil (tungsten coil) had an outer diameter of 0.342 mm, an inner diameter of 0.180 mm, and a length of 120 mm, and was formed by helically winding a tungsten wire with a diameter of 0.081 mm.

The tungsten wire was subjected to a roughening process (NaOH aqueous solution, 600 degrees, 20 seconds) in advance.

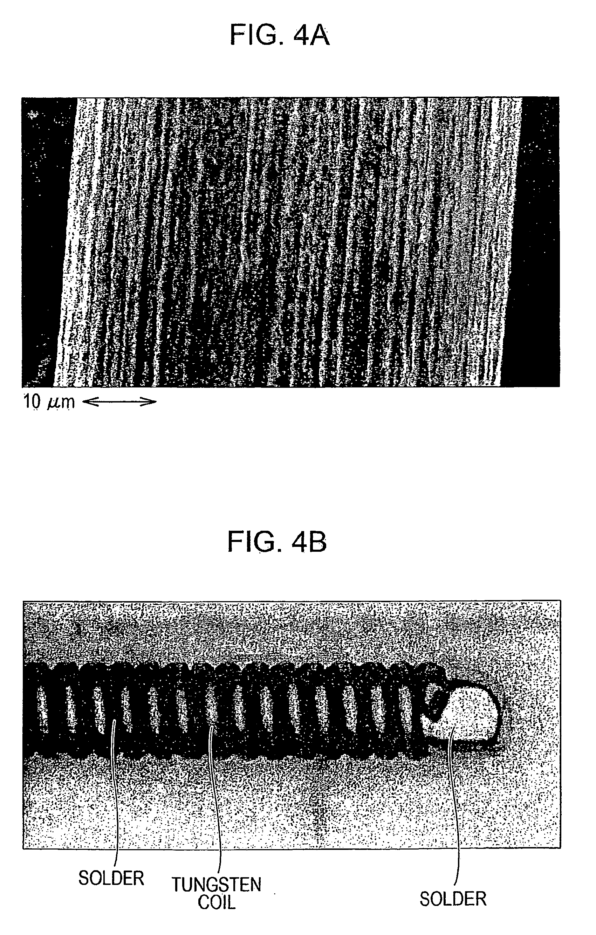

The roughened surface of the tungsten wire was observed with a scanning electron microscope at a magnification of 1,800. Fig. 4A is a scanning electron micrograph that shows an enlarged view of the tungsten wire forming the helical coil in the model of the guidewire according to the present invention.

As is clear from Fig. 4A, a plurality of grooves extending along the axial direction (vertical direction in Fig. 4A) of the tungsten wire were formed in the surface of the tungsten wire after the roughening process. Any grooves formed along the circumferential direction (horizontal direction in Fig. 4A) of the wire are not shown in Fig. 4A. However, more grooves were formed along the axial direction (vertical direction in Fig. 4A) of the wire than along the circumferential direction (horizontal direction in Fig. 4A) of the wire.

The manufacturing process of model 1 will be further explained. The prepared stainless steel rod was inserted into the helical coil. Then, the stainless steel rod and the helical coil were bonded together with the Sn-Ag alloy solder. Model 1 was manufactured by the above-described process.

The state of the solder parts of model 1 was observed. Fig. 4B is a macrophotograph of the front end portion of the model including the tungsten wire illustrated in Fig. 4A.

As illustrated in Fig. 4B, in Model 1, spaces between the adjacent portions of the wire in the helical coil (tungsten coil) were filled with the solder, and the solder did not protrude from the helical coil. Thus, it was confirmed that the helical coil was reliably bonded with the solder parts. Therefore, it can be presumed that the bonding strength between the helical coil and the solder parts is high.

### Comparative Example 1

Model 2 was manufactured by a process similar to that of Experimental Example 1 except the roughening process was not performed.

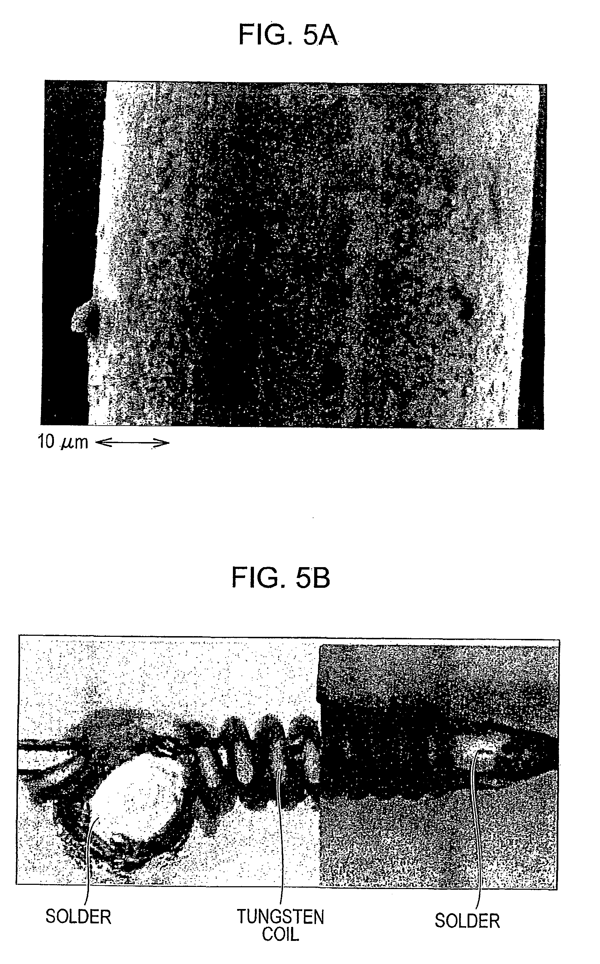

The surface of the tungsten wire that was not subjected to the roughening process was observed with a scanning electron microscope at a magnification of 1,800. Fig. 5A is a scanning electron micrograph that shows an enlarged view of the tungsten wire forming the helical coil in the model of the guidewire according to the related art.

As is clear from Fig. 5A, grooves are hardly formed in the surface of the tungsten wire that was not subjected to the roughening process.

The state of the solder parts of model 2 was observed. Fig. 5B is a macrophotograph of the front end portion of the model including the tungsten wire illustrated in Fig. 5A. In Fig. 5B, an intermediate portion of the helical coil is omitted and only the areas around the front and rear ends of the helical coil are shown.

As illustrated in Fig. 5B, in Model 2, spaces between the adjacent portions of the wire in the helical coil are not filled with the solder, and the solder largely protrudes from the helical coil. Therefore, it can be presumed that the bonding strength between the helical coil and the solder parts is low.

### Other Embodiments

In the guidewire according to the present invention, the plurality of grooves that may be formed such that more grooves are formed along the axial direction of the wire than along the circumferential direction of the wire may be provided over the entire area of the surface of the wire. Alternatively, the grooves may be provided only in the areas in which the helical coil is bonded with the solder parts.

In the guidewire according to the present invention, the helical coil may be tapered such that the diameter thereof decreases toward the front end from the rear end of the helical coil. The guidewire including a helical coil having such a shape is desirable since the guidewire can be easily inserted into a hard lesion, such as a chronic total occlusion lesion.

The outer surface of the guidewire according to the present invention may be covered with a hydrophilic material. In such a case, the guidewire can be smoothly moved through a guiding catheter, a tube, or a body tissue by reducing the sliding friction.

Examples of the hydrophilic material include cellulose-based high-polymer materials, polyethylene-oxide-based high-polymer materials, maleic-anhydride-based high-polymer materials (e.g., maleic anhydride copolymers such as methyl vinyl ether-maleic anhydride copolymers), acrylamide-based high-polymer materials (e.g., polyacrylamides and polyglycidyl methacrylate-dimethylacrylamide (PGMA-DMAA) block copolymers), water-soluble nylons, polyvinyl alcohols, polyvinyl pyrrolidones, and hyaluronates. In particular, hyaluronates are preferably used.

## Claims

1. A guidewire (1) comprising:
a core shaft (2) having a front end portion (2a) and a rear end portion (2b);
a helical coil (3) in which the front end portion (2a) is inserted; and
a solder part with which the front end portion (2a) and the helical coil (3) are bonded to each other, **characterized in that**
the helical coil (3) comprises a wire (3a) having a plurality of grooves (3b) formed in a surface of the wire (3a) along an axial direction of the wire (3a).

2. The guidewire (1) according to Claim 1, wherein the plurality of grooves (3b) formed in the surface of the wire (3a) includes grooves formed along an axial direction of the wire (3a) and grooves formed along a circumferential direction of the wire (3a).

3. The guidewire (1) according to Claim 2, wherein more grooves (3b) are formed along the axial direction of the wire (3a) than along the circumferential direction of the wire (3a).

4. The guidewire (1) according to one of Claims 1 to 3, wherein the wire (3a) comprises tungsten.

## Patentansprüche

1. Führungsdraht (1) mit:
einem Kernschaft (2) mit einem vorderen Endabschnitt (2a) und einem hinteren Endabschnitt (2b);
einer Schraubenfeder (3), in der der vordere Endabschnitt (2a) eingesetzt ist; und
einer Löststelle, an der der vordere Endabschnitt (2a) und die Schraubenfeder (3) aneinandergefügt sind, **dadurch gekennzeichnet, dass**
die Schraubenfeder (3) einen Draht (3a) mit einer Vielzahl von Nuten (3b) aufweist, die in Axialrichtung des Drahts (3a) an dessen Oberfläche ausgebildet sind.

2. Führungsdraht (1) nach Anspruch 1, wobei die Vielzahl von Nuten (3b), die an der Oberfläche des Drahts (3a) ausgebildet sind, in Axialrichtung des Drahts (3a) ausgebildete Nuten und in Umfangsrichtung des Drahts (3a) ausgebildete Nuten umfassen.

3. Führungsdraht (1) nach Anspruch 2, wobei in Axialrichtung des Drahts (3a) mehr Nuten (3b) als in Umfangsrichtung des Drahts (3a) ausgebildet sind.

4. Führungsdraht (1) nach einem der Ansprüche 1 bis 3, wobei der Draht (3a) Wolfram enthält.

## Revendications

1. Fil-guide (1) comprenant :
un axe central (2) ayant une portion d'extrémité avant (2a) et une portion d'extrémité arrière (2b) ;
une bobine hélicoïdale (3) dans laquelle la portion d'extrémité avant (2a) est insérée ; et
une partie de brasure avec laquelle la portion d'extrémité avant (2a) et la bobine hélicoïdale (3) sont liées l'une à l'autre, **caractérisé en ce que**
la bobine hélicoïdale (3) comprend un fil (3a) ayant une pluralité de rainures (3b) formées dans une surface du fil (3a) le long d'une direction axiale du fil (3a).

2. Fil-guide (1) selon la revendication 1, dans lequel la pluralité de rainures (3b) formées dans la surface du fil (3a) comprend des rainures formées le long d'une direction axiale du fil (3a) et des rainures formées le long d'une direction circonférentielle du fil (3a).

3. Fil-guide (1) selon la revendication 2, dans lequel davantage de rainures (3b) sont formées le long de la direction axiale du fil (3a) que le long de la direction circonférentielle du fil (3a).

4. Fil-guide (1) selon l'une quelconque des revendications 1 à 3, dans lequel le fil (3a) comprend du tungstène.
